# EUROPEAN PATENT SPECIFICATION

(11) **EP 2 463 022 B1**
(45) Date of publication and mention of the grant of the patent: **04.04.2018**
(21) Application number: 10806528.5
(22) Date of filing: 05.08.2010
(51) Int. Cl.: B01J 13/00, A01M 1/20, A01N 25/02, A01N 43/78, A01P 3/00, A61K 8/22, A61L 9/04, A61Q 13/00, B01F 3/04, B01F 3/08, B01F 17/00, C11B 9/00, C11D 7/24, C11D 17/00, C11D 17/08

(54) **COMPOSITION AND PROCESS FOR PRODUCTION THEREOF**
ZUSAMMENSETZUNG UND HERSTELLUNGSVERFAHREN DAFÜR
COMPOSITION ET SON PROCÉDÉ DE FABRICATION

(30) Priority: 06.08.2009 JP 2009183755
(43) Date of publication of application: 13.06.2012
(73) Proprietor: LIGARIC CO., LTD., Osaka 565-0805 (JP); Sunstar Giken Kabushiki Kaisha, Takatsuki-shi, Osaka 569-1134 (JP)
(72) Inventor: TSUJI, Hideyasu, Fukuyama-shi Hiroshima 721-0955 (JP); TSUJI, Yasuhiro, Fukuyama-shi Hiroshima 721-0955 (JP); OKA, Toru, Takatsuki-shi Osaka 569-1134 (JP); SUGI, Shigeru, Takatsuki-shi Osaka 569-1134 (JP); TORII, Masumi, Takatsuki-shi Osaka 569-1134 (JP); MIYAO, Haruka, Takatsuki-shi Osaka 569-1134 (JP); NAKAYAMA, Yoshimitsu, Takatsuki-shi Osaka 569-1134 (JP); TORII, Tomoyuki, Takatsuki-shi Osaka 569-1134 (JP); MORI, Masahito, Takatsuki-shi Osaka 569-1134 (JP)
(74) Representative: Wilkinson, Stephen John
(86) International application number: PCT/JP2010/063316
(87) International publication number: WO 2011/016529

(56) References cited:
- EP-A1- 2 545 974
- WO-A1-2007/004274
- WO-A1-2008/072371
- JP-A- 2005 246 294
- JP-A- 2006 241 243
- JP-A- 2007 161 977
- JP-A- 2008 279 424
- JP-A- 2008 296 095
- JP-A- 2009 136 852
- JP-A- 2010 189 318
- US-A1- 2006 054 205
- US-A1- 2007 189 972
- US-A1- 2008 220 089
- CAVALLI R ET AL: "Preparation and characterization of dextran nanobubbles for oxygen delivery", INTERNATIONAL JOURNAL OF PHARMACEUTICS, ELSEVIER BV, NL, vol. 381, no. 2, 17 July 2009 (2009-07-17) , pages 160-165, XP026643521, ISSN: 0378-5173, DOI: 10.1016/J.IJPHARM.2009.07.010 [retrieved on 2009-07-17]
- CHO S H ET AL: "Ultrasonic formation of nanobubbles and their zeta-potentials in aqueous electrolyte and surfactant solutions", COLLOIDS AND SURFACES. A, PHYSICACHEMICAL AND ENGINEERING ASPECTS, ELSEVIER, AMSTERDAM, NL, vol. 269, no. 1-3, 16 August 2005 (2005-08-16), pages 28-34, XP027802898, ISSN: 0927-7757 [retrieved on 2005-11-01]
- KUKIZAKI M ET AL: "Size control of nanobubbles generated from Shirasu-porous-glass (SPG) membranes", JOURNAL OF MEMBRANE SCIENCE, ELSEVIER SCIENTIFIC PUBL.COMPANY. AMSTERDAM, NL, vol. 281, no. 1-2, 15 September 2006 (2006-09-15), pages 386-396, XP024931886, ISSN: 0376-7388, DOI: 10.1016/J.MEMSCI.2006.04.007 [retrieved on 2006-09-15]

## Description

### FIELD OF THE INVENTION

The present invention relates to a composition comprising a large amount of ultrafine bubbles and a drug which is an evaporative substance selected from fragrances and essential oils, a dispersion having a hydrophobic drug dispersed in water in absence of a surfactant and processes for producing the composition and the dispersion.

### BACKGROUND OF THE INVENTION

Recently, an apparatus has been developed that generates ultrafine bubbles commonly called nanobubbles. However, the use of this apparatus is limited to those applications where water that contains nanobubbles is used in washing operations or wastewater treatment and no studies have been made concerning drug-containing systems.

A method in which a chemical substance is used in combination, not with nanobubbles, but with bubbles of a comparatively large diameter is known being disclosed in JP 2008-238165A. The invention disclosed in this patent application relates to a dispersing method for keeping stable a dispersion having a substance dispersed in a liquid, which is characterized by incorporating bubbles in the dispersion. However, the main thrust of this method is that a dispersion having improved stability is produced by causing bubbles to be present in the process of its production and the resulting dispersion has no bubbles present in it. This should be clear from the fact that in the invention disclosed in JP 2008-238165A, the preferred diameter of the bubbles to be used ranges from 30 to 1000 microns but that bubbles of 1000 microns (1 mm) are unable to exist stably in the dispersion for an extended period of time. In addition, the particle size of the bubbles greatly differs from that of the ultrafine bubbles to be used in the present invention and the effect of such bubbles is by no means satisfactory, as demonstrated by the Examples in which more than 10% of the dispersed oil separated in 48 hours.

### RELATED LITERATURE

### Patent Literature

Patent Document 1: JP 2008-238165A

### SUMMARY OF THE INVENTION

### Problem to be Solved by the Invention

The present inventor found that a composition comprising novel ultrafine bubbles in the nano-range and a drug allowed the drug to exhibit its effect more pronouncedly and that when the drug was dispersed, a stable dispersion could be obtained without using a surfactant; the present invention has been accomplished on the basis of these findings.

The present invention relates to a composition comprising ultrafine bubbles having a mode particle size of no more than 500nm,; a drug; and water, wherein the ultrafine bubbles are present at a density of at least 1 x 10⁶ per millilitre, wherein the surfaces of the ultrafine bubbles contained in the composition are electrically charged to provide zeta potentials that are at least 5 mV in absolute value, and wherein the drug is an evaporative substance selected from fragrances and essential oils. The present invention also provides a process for preparing the composition of the invention comprising
generating by means of an ultrafine bubble generator ultrafine bubbles in water which ultrafine bubbles have a mode particle size of no more than 500nm and which are present at a density of at least 1 x 10⁶ per millilitre, wherein the surfaces of the ultrafine bubbles are electrically charged to provide zeta potentials that are at least 5mV in absolute value,
wherein a drug which is an evaporative substance selected from fragrances and essential oils is dissolved or dispersed in the water prior to the generation of the ultrafine bubbles or is dispersed in the water after the generation of the ultrafine bubbles.

### Means for Solving the Problem

The present invention provides a composition comprising ultrafine bubbles having a mode particle size of no more than 500nm; a drug; and water, wherein the ultrafine bubbles are present at a density of at least 1 x 10⁶ per millilitre, wherein the surfaces of the ultrafine bubbles contained in the composition are electrically charged to provide zeta potentials that are at least 5mV in absolute value, and wherein the drug is an evaporative substance selected from fragrances and essential oils.

In a first mode of the present invention, the drug is a water-soluble drug and dissolved in water.

In a second mode of the present invention, the drug is a hydrophobic drug and dispersed in the water. To be more specific, the hydrophobic drug is dispersed as dispersoid particles in the water which serves as the dispersion medium.

In the second mode of the present invention, mode particle size of the dispersed drug particles preferably ranges from 0.05 µm to 15 µm. The mean particle size of the dispersed drug particles may also preferably range from 0.05 µm to 15 µm. Depending on the type of the hydrophobic drug to be dispersed, there can be formed such fine drug particles that their mode particle size and/or mean particle size ranges from 0.05 µm to 3 µm. Note that the "hydrophobic drug" as used herein refers to a drug that is poorly soluble in water but which is oil-soluble.

The aforementioned ultrafine bubbles have a mode particle size of no more than 500 nm, preferably no more than 300 nm, and most preferably no more than 150 nm, and they are present at a density of at least 1 x 10⁶, preferably at least 3 x 10⁶, more preferably at least 4 x 10⁶, and most preferably at least 5 x 10⁶ bubbles, per millilitre.

According to the invention, the surfaces of the ultrafine bubbles contained in the composition or dispersion are electrically charged to provide zeta potentials that are at least 5 mV in absolute value.

The drug is an evaporative substance selected from fragrances and essential oils.

The composition or dispersion of the present invention need not be a liquid and may instead be in a gel form. To render the dispersion into a gel form, agar, carrageenan, gelatin, water absorbing resins, aqueous polymers, etc. may be used. For example, carrageenan is added to distilled water and the mixture is heated to prepare a carrageenan solution, which is mixed well under agitation with the composition comprising the fine bubbles, drug and water. The resulting mixture is cooled to room temperature to thereby form a gelled dispersion. If desired, the dispersion may be converted to a mist by using an atomizer.

A composition comprising ultrafine bubbles having a mode particle size of no more than 500 nm, water and a water-soluble drug dissolved in water is prepared by a process wherein ultrafine bubbles having a mode particle size of no more than 500 nm is generated in a solution of the water-soluble drug and water by means of an ultrafine bubble generator.

A composition comprising ultrafine bubbles having a mode particle size of no more than 500 nm, water and a hydrophobic drug dispersed in water is prepared by a process wherein ultrafine bubbles having a mode particle size of no more than 500 nm is generated in a dispersion of water and the hydrophobic drug by means of an ultrafine bubble generator.

A composition comprising ultrafine bubbles having a mode particle size of no more than 500 nm, water and a hydrophobic drug dispersed in water may alternatively be prepared by a process wherein ultrafine bubbles having a mode particle size of no more than 500 nm is generated in water by means of an ultrafine bubble generator and, after that, the hydrophobic drug is added to water comprising the ultrafine bubbles.

### Advantageous Effects of Invention

Since it contains the ultrafine bubbles, the composition of the present invention allows the drug to exhibit its effect more pronouncedly. For example, the evaporation of the evaporative substance is improved and its concentration in the composition can be reduced accordingly. Conventionally, evaporation has been accomplished by various methods including thermal evaporation, volatilization of the drug by wind or by evaporation by an ultrasonic vibrator. However, the thermal method has the disadvantage of not permitting the use of substances that have a tendency to decompose with heat and, in addition, since the respective methods use a heating device, a fan, and an ultrasonic vibrator, they all suffer from an increased cost of manufacturing the evaporation apparatus and entail the operating cost. In contrast, the present invention is not only safe and low in the cost of manufacturing the apparatus but also requires no operating cost, with the additional advantage of being applicable to a wide range of substances in a safe manner.

If a hydrophobic drug is dispersed in water, the present invention offers the advantage of providing a dispersion that remains stable for an extended period of time without using a surfactant. Since no surfactant is used, cost reduction is possible and there is no need for treating waste liquid that would otherwise occur if a surfactant were used. Particularly in the case where the diameter of particles is decreased in order to improve the stability of the dispersion, it has been necessary to use a large amount of surfactant; in the present invention, however, there is no need to use a surfactant, so not only further cost reduction is achieved but also the problem of a decrease in the available content of the actually dispersed substance on account of the increased use of a surfactant can be solved.

### BRIEF DESCRIPTION OF THE DRAWINGS

FIG. 1 shows the size distribution of freshly generated ultrafine bubbles for use in the present invention and the change in it until after the lapse of 3 months (as measured with Multisizer 3).
FIG. 2 shows the result of measurement of the particle diameter of a sample of the ultrafine bubbles to be used in the present invention (as measured with the nanoparticle size analyzing system: NanoSight Series).
FIG. 3 shows the result of measurement of the particle diameter of another sample of the ultrafine bubbles to be used in the present invention (as measured with the nanoparticle size analyzing system: NanoSight Series).
FIG 4 shows the result of measurement of the zeta potential on the ultrafine bubbles to be used in the present invention (as measured with ELSZ-1 of OTSUKA ELECTRONICS CO., LTD.)
FIG. 5 shows graphically the particle size distribution of the emulsion as freshly prepared in Example 2 (the measurement conducted with the particle size distribution analyzer LS 13 320).
FIG. 6 shows graphically the particle size distribution of the emulsion that was prepared in Example 2 and stored at room temperature for 3 months (the measurement conducted with the particle size distribution analyzer LS 13 320).
FIG. 7 shows graphically the particle size distribution of the emulsion that was prepared in Example 2 and stored at 40 °C for 3 months (the measurement conducted with the particle size distribution analyzer LS 13 320).
FIG. 8 shows graphically the particle size distribution of the dispersion as freshly prepared in Example 3 (the measurement conducted with the particle size distribution analyser LS 13 320).
FIG. 9 shows graphically the particle size distribution of the dispersion that was prepared in Example 3 and stored at room temperature for 2 months (the measurement conducted with the particle size distribution analyser LS 13 320).
FIG. 10 shows graphically the particle size distribution of the dispersion that was prepared in Example 3 and stored at 40°C for 2 months (the measurement conducted with the particle size distribution analyzer LS 13 320).
FIG. 11 shows graphically the particle size distribution of the dispersion as freshly prepared in Example 4 (the measurement conducted with the particle size distribution analyzer LS 13 320).
FIG. 12 shows graphically the particle size distribution of the dispersion that was prepared in Example 4 and stored at room temperature for 2 months (the measurement conducted with the particle size distribution analyzer LS 13 320).
FIG. 13 shows graphically the particle size distribution of the dispersion that was prepared in Example 4 and stored at 40°C for 2 months (the measurement conducted with the particle size distribution analyzer LS 13 320).

### DETAILED DESCRIPTION OF THE INVENTION

The present invention provides a composition according to claim 1, comprising ultrafine bubbles having a mode particle size of no more than 500 nm and a drug which is an evaporative substance selected from fragrances and essential oils, as well as water.

The particle diameter of the ultrafine bubbles to be used in the present invention is so small that it cannot be measured correctly with an ordinary particle size distribution analyzer. Hence, hereinafter, numerical values are employed that were obtained by measurements with the nanoparticle size analyzing system NanoSight Series (product of NanoSight Ltd.). The nanoparticle size analyzing system NanoSight Series (product of NanoSight Ltd.) measures the velocity of nanoparticles moving under Brownian motion and calculates the diameters of the particles from the measured velocity. A mode particle size can be verified from the size distribution of the particles present. The interior of the ultrafine bubbles is generally filled with air, which may be replaced by other gases including oxygen, hydrogen, nitrogen, carbon dioxide, and ozone.

The drug is an evaporative substance selected from fragrances and essential oils and may be any compound that works effectively for a desired object. From the aspect of chemical structure, the drug may be exemplified by but are not limited to water-soluble substances such as various water-soluble natural substances, as well as hydrophobic substances such as plant-derived oils, animal-derived oils, lipids, hydrocarbons, and high alcohols.

Water that can be used may be exemplified by distilled water, ultrapure water, highly pure water, pure water, tap water, ion-exchanged water deionized water, filtered water, electrolyzed water, and natural water. If performance is not compromised, a water-miscible solvent such as alcohol may be contained as a co-solvent in a small quantity.

In the first mode of the present invention, the above-described drug is dissolved in water. Water-soluble drugs to be used in this mode include, for example, fragrances. Exemplary water-soluble drugs include the following: water-soluble plant extracts including rosemary extract, lemon extract, Litchi chinensis extract, Momordica charantia var. pavel extract, star flute extract, Alpinia zerumbet extract, Ginkgo biloba extract, kaki (Japanese persimmon) extract, lavender extract, wormwood extract, peach leaf extract, sage extract, pine extract, Luffa cylindrica (L.) Roem. extract, carrot extract, Angelica acutiloba root extract, tomato extract, red pepper extract, aloe extract, seaweed extract, sage extract, Eugena caryophyllus (clove) flower extract, corn maize extract, thyme extract, eucalyptus leaf extract, Cupressus sempervirens extract, savory extract, clove extract, mint extract, pepper extract, tea extract, Rosa roxburghii extract, and sugar cane liquid extract; some of these extracts may be used in combination. It should be mentioned that the foregoing examples are non-limiting and the scope of the present invention is by no means limited to those compounds.

In the second mode of the present invention, the aforementioned drug is dispersed in water.

In this mode, the drug forms a discontinuous phase as the dispersoid whereas water forms a continuous phase as the dispersion medium. The hydrophobic drugs to be used in this mode are fragrances (air fresheners) and essential oils. Exemplary hydrophobic drugs include the following: acetyl-iso-eugenol, anethole, iso-amyl acetate, allylamyl glycolate, allyl heptanoate, aldehyde C-14 peach, aldehyde C-16 strawberry, estragole, eugenol, 1-carvone, camphor, camphene, iso-cyclocitral, 1,8-cineole, citral, citronellal, dimetol, dimethyl benzyl carbinyl acetate, α-damascone, β-damascone, δ-damascone, damascenone, terpineol, terpinyl acetate, terpinolene, terpinen-4-ol, thymol, o-t-butylcyclohexyl acetate, cis-3-hexenyl acetate, FRUITATE, POIRENATE, POLLENAL II, iso-bornyl acetate, p-methyl acetophenone, methyl-iso-eugenol, methyl ionone-γ, 1-menthol, menthone, iso-menthone, methyl salicylate, menthanyl acetate, lactone C-10 gamma, linalyl acetate, aldehyde C-11, aldehyde C-12 lauric, aldehyde C-12 MNA, ambroxan, amylcinnamic aldehyde, amyl salicylate, benzaldehyde, benzyl acetate, benzyl salicylate, cedrol, cinnamic alcohol, coumarin, cyclopentadecanolide, γ-decalactone, ethyl vanillin, eugenol, hexylcinnamic aldehyde, indole, α-ionone, isoeugenol, lilial, linalool, linalyl acetate, lyral, maltol, methyl anthranilate, methylionone, γ-methylionone, musk ketone, musk xylol, phenyl acetaldehyde, phenyl acetate, phenylethyl alcohol, phenylpropyl alcohol, α-pinene, α-terpineol, tonalid, vanillin, and Vertofix Coeur, as well as essential oils including rosemary oil, lemon grass oil, mint oil, spearmint oil, sage oil, ginger oil, anise oil, armoise oil, estragon oil, cardamom oil, camphor oil, caraway oil, carrot seed oil, clove oil, coriander oil, citronella oil, spearmint oil, clary sage oil, thyme oil, pine oil, basil oil, fennel oil, volatile laurel oil, peppermint oil, lavandine oil, marjoram oil, lavender oil, laurel leaf oil, eucalyptus oil, and neem oil; oil-soluble plant extracts including tea extract, Rosa roxburghii extract, sugar cane extract, lemon extract, Litchi chinensis extract, Momordica charantia var. pavel extract, glucosamine, star fruit extract, Alpinia zerumbet extract, Ginkgo biloba extract, fruit juice, kaki (Japanese persimmon) extract, lavender extract, wormwood extract, peach leaf extract, sage extract, pine extract, Luffa cylindrica (L.) Roem. extract, carrot extract, Angelica acutiloba root extract, tomato extract, red pepper extract, aloe extract, seaweed extract, sage extract, Eugena caryophyllus (clove) flower extract, corn maize extract, thyme extract, eucalyptus leaf extract, Cupressus sempervirens extract, savory extract, clove extract, mint extract, and pepper extract; as well as terpenes including terpene hydrocarbon such as pinene, menthene, cymene, phellandrene, menthane and limonene, and terepene alcohols such as citronellol, pinocampheol, gellaniol, fencyl alcohol, nerol, linalool, and borneol; some of these extracts may be used in combination. It should be mentioned that the foregoing examples are non-limiting and the scope of the present invention is by no means limited to those compounds.

If the hydrophobic drug is to be dispersed in water, the mode particle size of the drug particles preferably ranges from 0.05 µm to 15 µm, more preferably from 0.05 µm to 6 µm. Depending on the type of the drug to be dispersed, there can be formed ultrafine drug particles in the range of 0.05 µm to 3 µm. The mean size of the drug particles may also preferably range from 0.05 to 15 µm, more preferably from 0.05 µm to 6 µm. Depending on the type of the hydrophobic drug to be dispersed, there can be formed ultrafine drug particles having a mean size in the range of 0.05 µm to 3 µm.

The size distribution of the dispersed drug particles as referred to in the present invention was measured with the particle size distribution analyzer LS 13 320 (product of BECKMAN COULTER). The mode particle size is a maximum value of particle diameter as expressed in percentages by volume or number and is also called the mode particle diameter. The mean size is a number average diameter or volume average diameter. Note that the size distribution data to be shown later in the Examples are assumed to represent the size distributions of drug particles surrounded with ultrafine bubbles on the surface, and the ultrafine bubbles themselves.

In the present invention, ultrafine bubbles occur at a density of at least 1 x 10⁶, preferably at least 3 x 10⁶, more preferably at least 4 x 10⁶, and most preferably at least 5 x 10⁶, per millilitre. The number of ultrafine bubbles as referred to in the present invention was measured with the nanoparticle size analyzing system NanoSight Series (product of NanoSight Ltd.).

Although not wishing to be bound by theory, the present inventor assumes that the superior effects of the present invention are achieved by the following mechanism. If the drug is water-soluble, the moving ultrafine bubbles would enhance the motion of the drug molecules to make them more efficacious and the ultrafine bubbles would themselves increase the penetration of the aqueous solution to exhibit even better effect. If the drug is hydrophobic and dispersed in water, the ultrafine bubbles would gather on the surfaces of the dispersed drug particles and the zeta potential on the bubble surfaces would create a sufficient surface active effect to stabilize the dispersed particles. Therefore, it is important that the number of ultrafine particles be kept within a preferred range.

From the viewpoints just described above, the zeta potential on the surfaces of the ultrafine particles contained in the composition or dispersion is also considered to be important for ensuring the present invention to exhibit its intended effects. The surfaces of the ultrafine particles used in the present invention are electrically charged to produce a zeta potential of at least 5 mV, preferably at least 7 mV, in absolute value. Since the absolute value of zeta potential is proportional to the viscosity/dielectric constant of the solution, the lower the temperature at which the ultrafine bubbles, drug and water are treated, the more likely it is that the resulting dispersion has higher stability.

The ultrafine particles to be used in the present invention that have a mode particle size of no more than 500 nm can be generated by any known means, such as the use of a static mixer, the use of a venturi tube, cavitation, vapor condensation, sonication, swirl formation, dissolution under pressure, or fine pore formation. A preferred method of bubble generation is by forming a gas-liquid mixture and shearing it.

An advantageous apparatus for generating ultrafine bubbles by the gas-liquid mix shearing method is disclosed in Japanese Patent No. 4118939. In this apparatus, the greater part of a gas-liquid mixture in fluid form introduced into a fluid swirling compartment does not simply flow toward the discharge port as in the apparatus described in the prior art section but it first flows forming a swirl in the direction away from the discharge port. The swirl reaching the first end-wall member turns around and flows back toward the second end-wall member; since the returning swirl has a smaller radius of rotation than the swirl flowing toward the first end-wall member, it flows at a higher velocity, creating a sufficient shear force on the gas within the liquid to promote the formation of finer bubbles.

If the drug is water-soluble, its aqueous solution is treated with a suitable apparatus to generate ultrafine bubbles in it, whereby the composition of the present invention can be produced that has the drug dissolved in the water.

If the drug is hydrophobic, a mixture of the hydrophobic drug and water is treated with a suitable apparatus to generate ultrafine bubbles in the aqueous dispersion of the hydrophobic drug, whereby the composition of the present invention can be produced that has the hydrophobic drug dispersed in the water. Alternatively, water may be treated with a suitable apparatus to generate ultrafine bubbles in it and thereafter the hydrophobic drug is added, whereby the composition of the present invention can be produced that has the hydrophobic drug dispersed in the water. Note that a hydrophobic drug that is solid at ordinary temperature may also be used if it is thermally melted or dissolved in a solvent.

There is no need to use a surfactant in the present invention but it should be appreciated by skilled artisans that this does not mean excluding the case of adding a surfactant as appropriate for use and other conditions.

The foregoing description of the present invention and the description of the Examples that follow are only intended to provide a detailed explanation of various exemplary embodiments of the present invention and skilled artisans can made various improvements and changes of the embodiments disclosed herein without departing from the scope of the present invention. Therefore, the description herein will in no way limit the scope of the present invention, which shall be determined only by the recitation in the appended claims.

### EXAMPLES

### Example 1

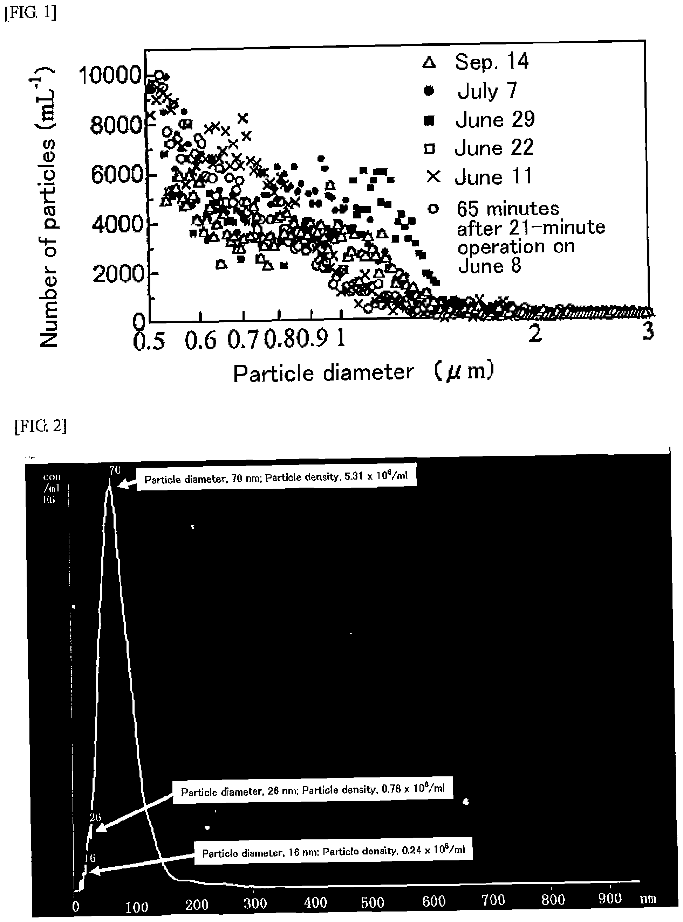

Ultrafine bubbles were generated in pure water having a resistivity of 18.2 MΩ/cm using BUVITAS of KYOWA KISETSU which was a device for generating ultrafine bubbles by the gas-liquid mix shearing method. FIG. 1 shows the size distribution of the freshly generated ultrafine bubbles and the change in it until after the lapse of 3 months. Size distribution was measured with Multisizer 3 (product of BECKMAN COULTER). Obviously, there was no change in the number of bubbles with diameters of no more than 1 µm.

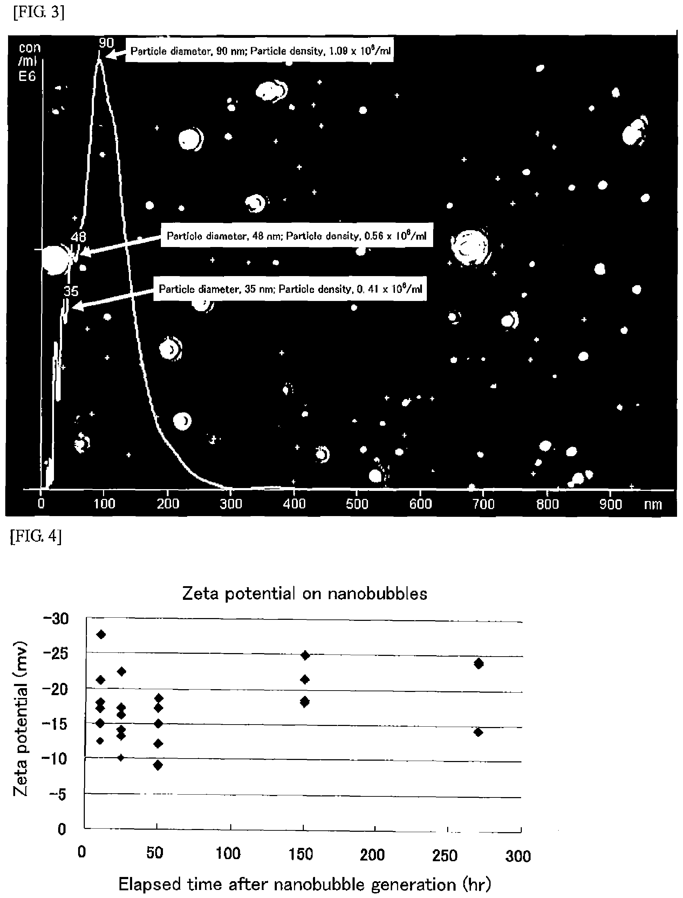

At the same time, the diameters of the generated ultrafine bubbles were measured with the nanoparticle size analyzing system NanoSight Series (product of NanoSight). The results are shown in FIGS. 2 and 3. The horizontal axis of each graph represents the particle diameter in nanometers and the vertical axis represents the number of particles per millimeter (1 x 10⁶/ml). FIG. 2 shows the result of a measurement that was conducted 24 hours after the generation of the ultrafine bubbles and FIG 3 shows the result after the passage of 48 hours. It was verified that the bubbles had a mode particle size of no more than 500 nm, with 4 to 8 x 10⁶ counts per ml, and that the generated ultrafine bubbles remained stable in the water for an extended period.

In addition, the zeta potential on the generated bubbles was measured with the zeta potential measuring system ELSZ-1 of OTSUKA ELECTRONICS CO., LTD. The result is shown in FIG. 4. Obviously, zeta potential was maintained for an extended period, indicating the stability of the bubbles.

### Examples 2-5

Using BUVITAS of KYOWA KISETSU, mixtures having the compositions shown in Table 1 below were treated under the same conditions that were used in Example 1, except that the pure water was replaced by distilled water. The results are shown in Table 1.

**Table 1**

| | | | Example 2 | Example 3 | Example 4 | Example 5 | Comp. Ex. 1 |
|---|---|---|---|---|---|---|---|
| Fine dispersion maker | | | Fine bubble generator having a gas-liquid mixture shearing devire*¹ | | | | Homomixer |
| Liquid dispersion | Distillad water | | 100 | 100 | 100 | 100 | 100 |
| | Oil compo -nent | Orange oil | 0.25 | | | | 0.25 |
| | | Neem extract | | 0.25 | | | |
| | | Limonene | | | 0.3 | | |
| Particle diameter µm | | just after preparation | 0.08 | 0.8 | 1.2 | 0.07 | 0.4 |
| | RT | 30 days | 0.80 | 0.9 | 1.2 | 0.07 | - |
| State of emulsion | | just aftre praparation | white translucent | white translucent | white translucent | transparent | separated |
| | RT | 2 weeks | do. | do. | do. | do. | do. |
| | | 30 days | do. | do. | do. | do. | do. |
| | | 60 days | do. | do. | do. | do. | do. |
| | | 90 days | do. | do. | do. | do. | do. |
| | 40°C | 30 days | do. | do. | do. | do. | do. |
| | | 60 days | do. | do. | do. | do. | do. |
| | | 90 days | do. | do. | do. | do. | do. |

| | | | | | | | |
|---|---|---|---|---|---|---|---|
| *1 Fine bubble generator BUVITAS of KYOWA KISETSU | | | | | | | |

Examples 2-4 showed that the hydrophobic drugs were stably dispersed. All samples, whether they were stored at room temperature (RT) or 40 °C, retained a satisfactory state of emulsification.

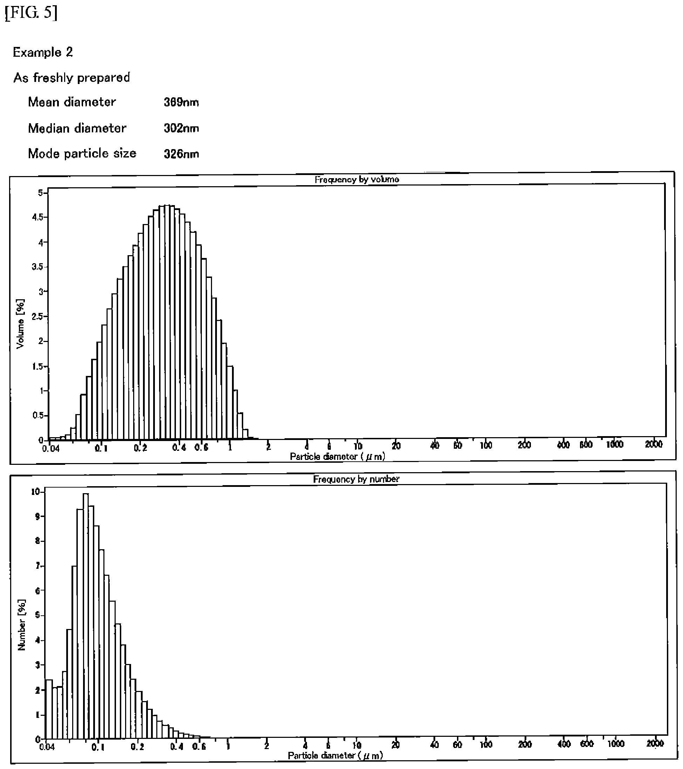

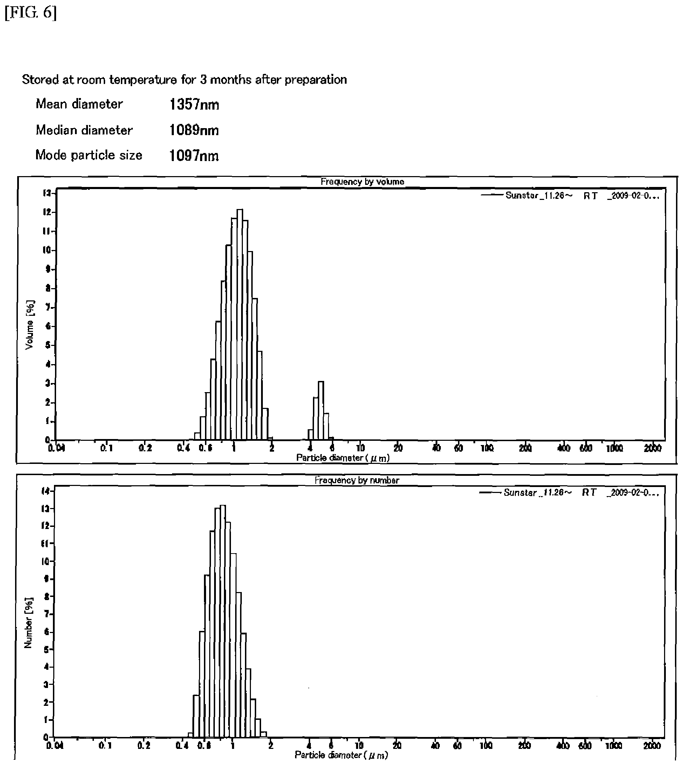

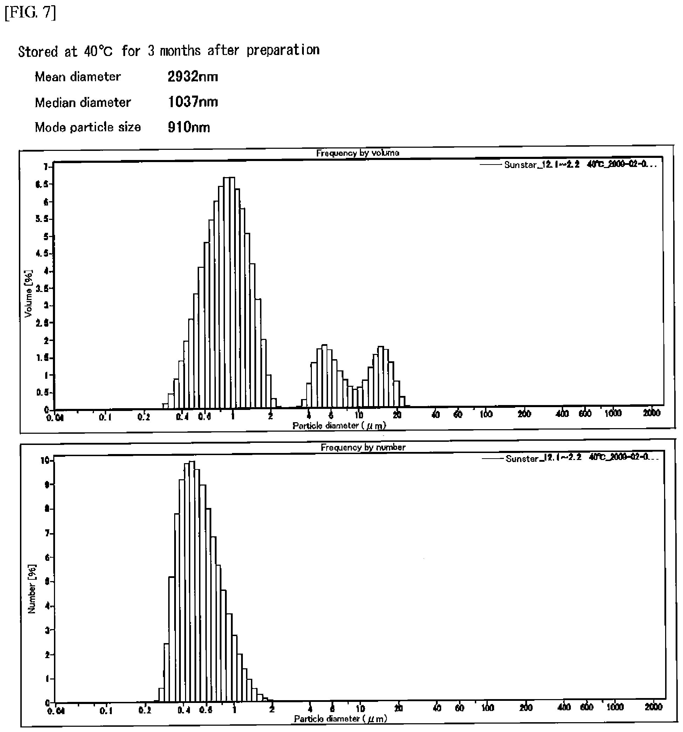

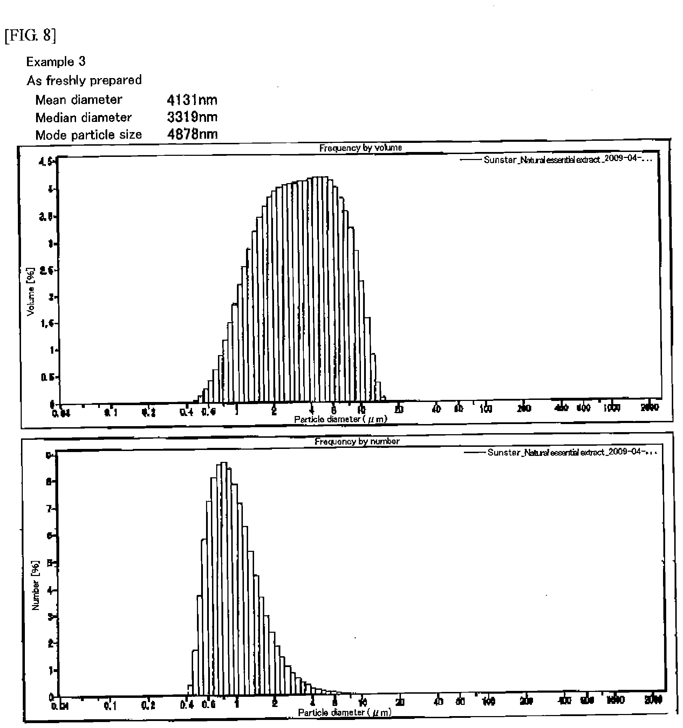

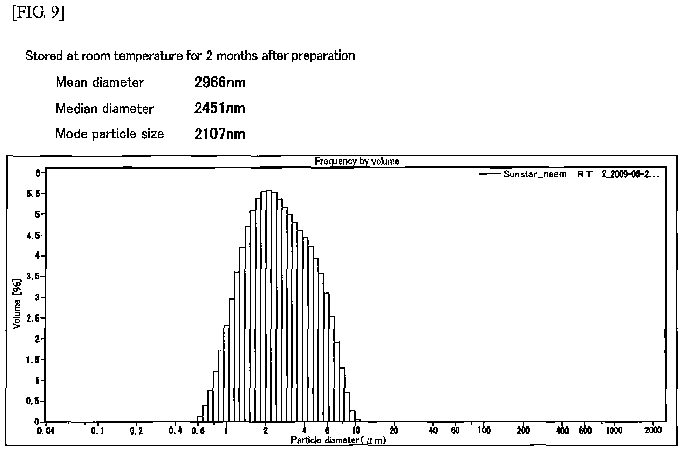

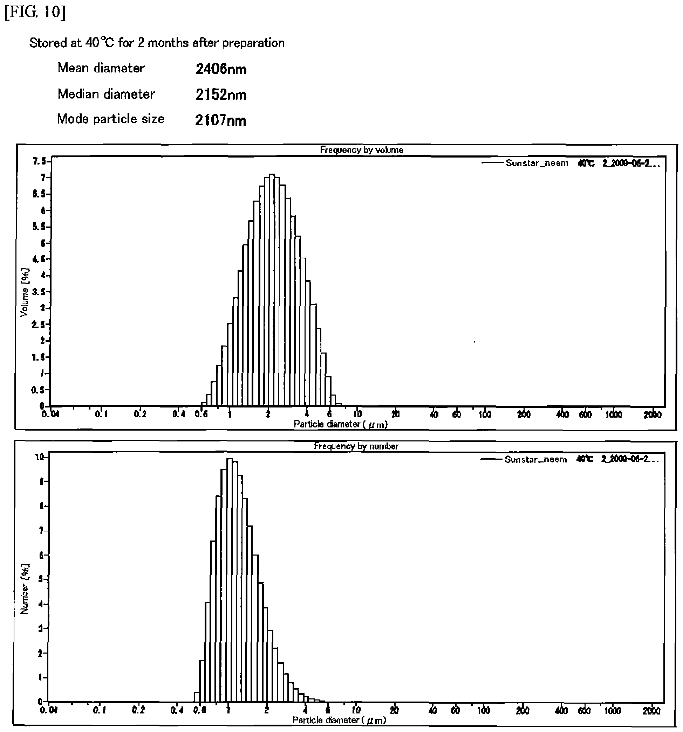

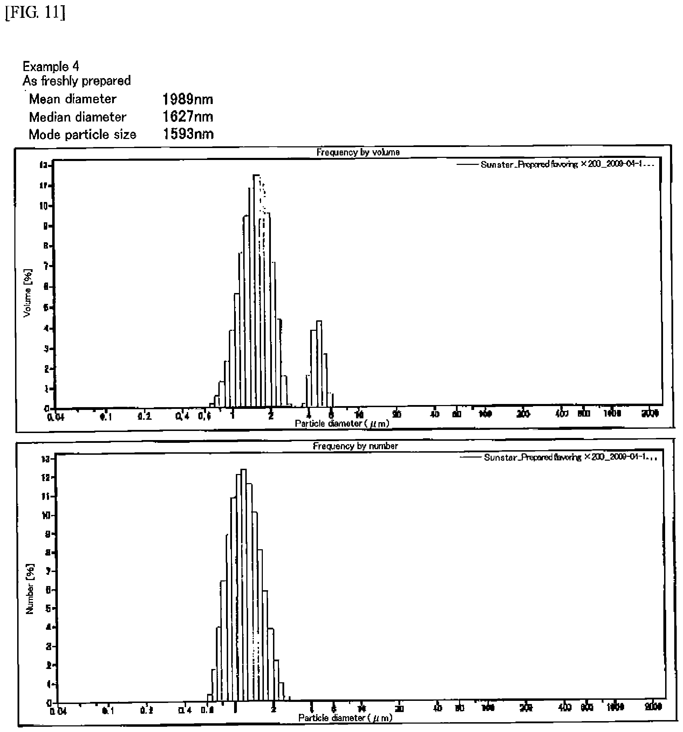

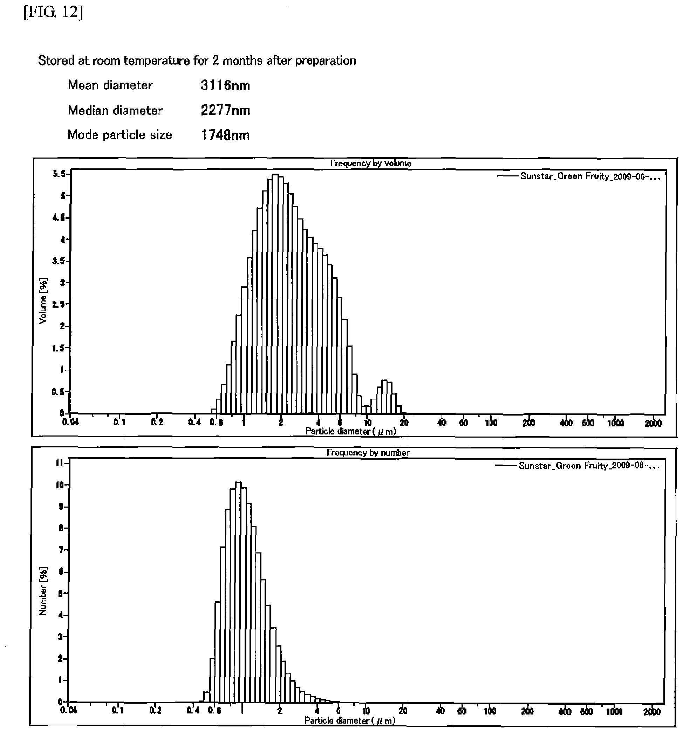

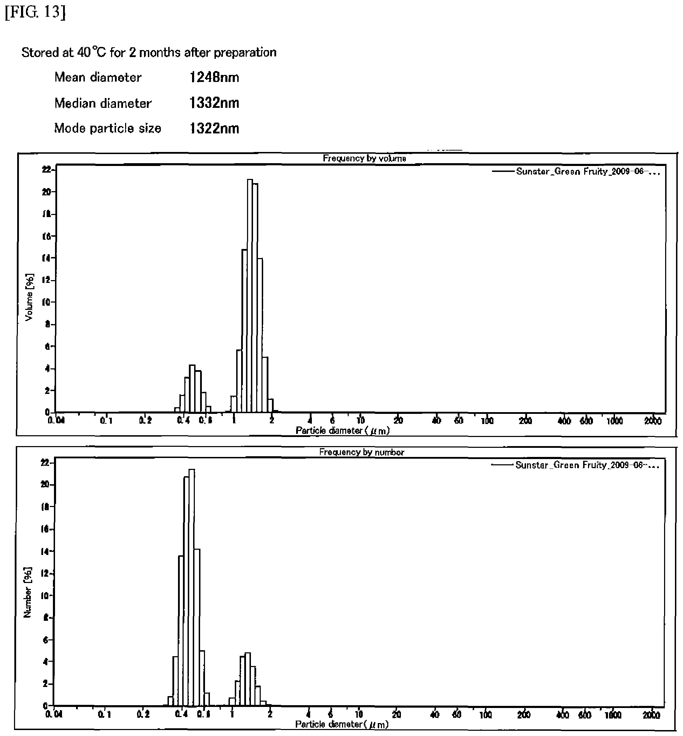

The particle size distributions of the dispersions prepared in Examples 2-4 were measured with the size distribution analyzer LS 13 320 (product of BECKMAN COULTER), both as freshly prepared and after storage at room temperature or 40 °C; the results are shown in FIGS. 5-13. The horizontal axis of each graph represents particle diameter and measurement was conducted for the volume and number percentages of each particle diameter, with the former being plotted in the upper panel and the latter in the lower panel. The values of mean size, median size and mode particle size in FIGS. 5-13 were calculated from volume percentages, and the data obtained from the sample of Example 3 after 2 month storage at room temperature were processed to measure only volume percentages. Although the particle diameter increased somewhat, the stability of dispersion was generally satisfactory in each of Examples 2-4.

As shown in Example 5, bubbles with a size of 70 nm were formed under the same experimental conditions. This would suggest that bubbles of approximately 70 nm in size were also formed in Examples 2-4.

As shown in Comparative Example 1, the dispersion prepared with a homomixer soon separated into two phases.

### Example 6

To prepare samples for Example 6, the components listed in Table 2 below were consecutively added in the amounts expressed in parts by weight in the same table and the mixtures were treated with BAVITAS or KYOWA KISETSU under the same conditions as in Example 1, except that the pure water was replaced by distilled water.

In Comparative Example 2, a surfactant was used to emulsify the same evaporative components as in Example 6.

**Table 2**

| | | | Example 6 | Comp. Ex. 2 |
|---|---|---|---|---|
| Fine dispersion maker | | | Fine bubble generator having a gas-liquid mixture shearing device *1 | Homomixer |
| Liquid dispersion. | Distilled water | | 100 | 100 |
| | Evaporative component | Limonene | 0.3 | 0.3 |
| | Surfactant | Tween | | 0.1 |
| Treatment conditions | | Time | 10 min | 2 min |
| | | Rotational speed (rpm) | - | 20000 |
| | | Temperature | 10-20°C | 10-20°C |

| | | | | |
|---|---|---|---|---|
| *1 Fine bubble generator: BAVITAS of KYOWA KISETSU | | | | |

### Examples 9 and 10

Mixtures having the compositions listed in Table 6 below were treated with BAVITAS of KYOWA KISETSU under the same conditions as in Example 1. In Example 10, deionized water was used after treatment with BAVITAS to generate ultrafine bubbles. Into the thus prepared ultrafine bubble-containing deionized water, 1-menthol was by means of a homomixer. In Comparative Examples 5 and 6, deionized water was also used but it was free from ultrafine bubbles; l-menthol was emulsified in this deionized water by means of a homomixer.

The results are shown in Table 6.

**Table 6**

| | | | Example 9 | Example 10 | Comp. Ex.5 | Comp. Ex. 6 |
|---|---|---|---|---|---|---|
| Ultrafine dispersion maker | | | Ultrafine bubble generator^{*1} | Homomixer^{*2} | Homomixer | Homomixer |
| Liquid dispersion | Deionized water | | | | | |
| | Hydrophobic component | l-menthol | 0.1 | 0.1 | 0.1 | 0.1 |
| | Surfactant | Polyoxyethylene casor oil | | | | 0.1 |
| Treatment conditions | Time | | 15 min | 15 min | 15 min | 15 min |
| | Rotational speed (rpm) | | - | 8000 | 8000 | 8000 |
| | Temperature | | RT | RT | RT | RT |
| Results of evaluation | State of emulsion (just after preparation) | | white translucent | white translucent | white translucent | white translucent |
| | State of emulsion (1 day after preparation) | | white translucent | white translucent | white translucent | white translucent |

| | | | | | | |
|---|---|---|---|---|---|---|
| *1 Fine bubble generator: BAVITAS of KUYOWA KISETSU *2 Deionized water containing ultrafine bubbles was used | | | | | | |

The sample of Example 9 maintained a more satisfactory state of emulsification than the sample of Comparative Example 5 which was simply treated with the homomixer. Good deionized water was also achieved in the sample of Example 10 which was prepared by adding l-menthol to the deionized water that was previously treated with BAVITAS of KYOWA KISETSU to generate ultrafine bubbles.

The compositions prepared in Examples 9 and 10 as well as Comparative Example 5 were evaluated for their masking performance.
Test method: In accordance with a modified odor bag method for odor sensory measurement, the samples identified in Table 6 (uniform dispersion just after preparation) were serially diluted with distilled water and subjected to sensory evaluation by 4 panelists for determining the thresholds of the samples. The procedure of threshold determination was the same as in Example 6. The results are shown in Table 7.

**Table 7**

| Sample | Panelist | Dilution ratio | | | | | Threshold | | |
|---|---|---|---|---|---|---|---|---|---|
| | | 1*10⁴ | 3*10⁴ | 1*10⁵ | 3*10⁵ | 1*10⁶ | | Average | Threshold ratio |
| | | 4.00 | 4.48 | 5.00 | 5.48 | 6.00 | | | |
| Example 9 | n=1 | ○ | ○ | ○ | X | - | 5.24 | 5.365 | 1*10^{5.37} |
| | n=2 | ○ | ○ | ○ | X | - | 5.24 | | |
| | n=3 | ○ | ○ | ○ | X | - | 5.24 | | |
| | n=4 | ○ | ○ | ○ | ○ | X | 5.74 | | |
| Example 10 | n=1 | ○ | ○ | X | - | - | 4.74 | 4.615 | 1*10^{4.62} |
| | n=2 | ○ | X | - | - | - | 4.24 | | |
| | n=3 | ○ | ○ | X | - | - | 4.74 | | |
| | n=4 | ○ | ○ | X | - | - | 4.74 | | |
| Comp. Ex.5 | n=1 | X | - | - | - | - | 3.74 | 4.115 | 1*10^{4.12} |
| | n=2 | ○ | X | - | - | - | 4.24 | | |
| | n=3 | ○ | X | - | - | - | 4.24 | | |
| | n=4 | ○ | X | - | - | - | 4.24 | | |

In Table 7, ○ means that the answer was correct and x means that the answer was wrong. The samples of Examples 9 and 10 had higher thresholds than the sample of Comparative Example 5, indicating an improvement in the efficiency of l-menthol's evaporation.

## Claims

1. A composition comprising ultrafine bubbles having a mode particle size of no more than 500nm;
a drug; and
water, wherein the ultrafine bubbles are present at a density of at least 1 x 10⁶ per millilitre,
wherein the surfaces of the ultrafine bubbles contained in the composition are electrically charged to provide zeta potentials that are at least 5 mV in absolute value,
and wherein the drug is an evaporative substance selected from fragrances and essential oils.

2. The composition according to claim 1, wherein the drug is dissolved in the water.

3. The composition according to claim 1, wherein the drug is dispersed in the water.

4. The composition according to claim 3, wherein the dispersed drug particles have a mode particle size in the range from 0.05 µm to 15 µm.

5. The composition according to claim 3, wherein the dispersed drug particles have a mean particle size in the range from 0.05 µm to 15 µm.

6. The composition according to any one of claims 1 to 5, wherein the composition is in a gel form.

7. The composition according to any one of claims 1 to 6, which contains at least one gas in the ultrafine bubbles as selected from the group consisting of oxygen, hydrogen, nitrogen, carbon dioxide, and ozone.

8. A process for preparing the composition of claim 1 comprising
generating by means of an ultrafine bubble generator ultrafine bubbles in water which ultrafine bubbles have a mode particle size of no more than 500nm and which are present at a density of at least 1 x 10⁶ per millilitre, wherein the surfaces of the ultrafine bubbles are electrically charged to provide zeta potentials that are at least 5mV in absolute value,
wherein a drug which is an evaporative substance selected from fragrances and essential oils is dissolved or dispersed in the water prior to the generation of the ultrafine bubbles or is dispersed in the water after the generation of the ultrafine bubbles.

9. A process according to claim 8, wherein the drug is water-soluble and the process comprises generating the ultrafine bubbles in a solution of the water-soluble drug in water.

10. A process according to claim 8, wherein the drug is hydrophobic and the process comprises generating the ultrafine bubbles in a dispersion of the hydrophobic drug in water.

11. A process according to claim 8, wherein the drug is hydrophobic and the process comprises adding the hydrophobic drug to water comprising the ultrafine bubbles after the generation of the ultrafine bubbles in the water.

12. A process according to any one of claims 8 to 11, wherein the ultrafine bubble generator is a gas-liquid mixture shearing device.

## Patentansprüche

1. Zusammensetzung, ultrafeine Bläschen mit einer Partikelgröße von maximal 500 nm;
einen Wirkstoff; und
Wasser umfassend, wobei die ultrafeinen Bläschen mit einer Dichte von mindestens 1 x 10⁶ pro Milliliter vorhanden sind,
wobei die Oberflächen der ultrafeinen Bläschen, die in der Zusammensetzung enthalten sind, elektrisch geladen sind, um Zetapotenziale zur Verfügung zu stellen, die mindestens 5 mV im absoluten Wert betragen,
und wobei der Wirkstoff ein aus Duftstoffen und essenziellen Ölen gewählter verdunstender Stoff ist.

2. Zusammensetzung nach Anspruch 1, wobei der Wirkstoff im Wasser gelöst ist.

3. Zusammensetzung nach Anspruch 1, wobei der Wirkstoff im Wasser dispergiert ist.

4. Zusammensetzung nach Anspruch 3, wobei die dispergierten Wirkstoffpartikel eine Partikelgröße im Bereich von 0,05 µm bis 15 µm aufweisen.

5. Zusammensetzung nach Anspruch 3, wobei die dispergierten Wirkstoffpartikel eine mittlere Partikelgröße im Bereich von 0,05 µm bis 15 µm aufweisen.

6. Zusammensetzung nach einem der Ansprüche 1 bis 5, wobei die Zusammensetzung in Gelform vorliegt.

7. Zusammensetzung nach einem der Ansprüche 1 bis 6, die mindestens ein Gas in den ultrafeinen Bläschen enthält, das aus der Gruppe bestehend aus Sauerstoff, Wasserstoff, Stickstoff, Kohlendioxid und Ozon gewählt wird.

8. Verfahren zum Herstellen der Zusammensetzung nach Anspruch 1, umfassend:
Erzeugen von ultrafeinen Bläschen in Wasser mithilfe eines Erzeugers ultrafeiner Bläschen, wobei die ultrafeinen Bläschen eine Partikelgröße von maximal 500 nm aufweisen und diese mit einer Dichte von mindestens 1 x 10⁶ Milliliter vorhanden sind, wobei die Oberflächen der ultrafeinen Bläschen elektrisch geladen sind, um Zetapotenziale zur Verfügung zu stellen, die mindestens 5 mV im absoluten Wert betragen,
wobei ein Wirkstoff, der ein aus Duftstoffen und essenziellen Ölen gewählter verdunstender Stoff ist, vor dem Erzeugen der ultrafeinen Bläschen im Wasser gelöst oder dispergiert wird oder nach dem Erzeugen der ultrafeinen Bläschen im Wasser dispergiert wird.

9. Verfahren nach Anspruch 8, wobei der Wirkstoff wasserlöslich ist und das Verfahren das Erzeugen der ultrafeinen Bläschen in einer Lösung des wasserlöslichen Wirkstoffs in Wasser umfasst.

10. Verfahren nach Anspruch 8, wobei der Wirkstoff hydrophob ist und das Verfahren das Erzeugen der ultrafeinen Bläschen in einer Dispersion des hydrophoben Wirkstoffs in Wasser umfasst.

11. Verfahren nach Anspruch 8, wobei der Wirkstoff hydrophob ist und das Verfahren das Hinzufügen des hydrophoben Wirkstoffs zu Wasser umfasst, das die ultrafeinen Bläschen nach dem Erzeugen der ultrafeinen Bläschen im Wasser enthält.

12. Verfahren nach einem der Ansprüche 8 bis 11, wobei der Erzeuger der ultrafeinen Bläschen eine Schervorrichtung für das Gas-Flüssigkeits-Gemisch ist.

## Revendications

1. Composition comprenant des bulles ultrafines ayant une dimension de particule modale ne dépassant pas 500 nm ;
un médicament ; et
de l'eau, dans laquelle les bulles ultrafines sont présentes à une densité d'au moins 1 x 10⁶ par millilitre,
dans laquelle les surfaces des bulles ultrafines contenues dans la composition sont chargées électriquement pour fournir des potentiels zêta qui sont d'au moins 5 mV en valeur absolue,
et dans laquelle le médicament est une substance évaporative sélectionnée parmi des parfums et des huiles essentielles.

2. Composition selon la revendication 1, dans laquelle le médicament est dissous dans l'eau.

3. Composition selon la revendication 1, dans laquelle le médicament est dispersé dans l'eau.

4. Composition selon la revendication 3, dans laquelle les particules de médicament dispersé ont une dimension de particule modale de l'ordre de 0,05 pm à 15 pm.

5. Composition selon la revendication 3, dans laquelle les particules de médicament dispersé ont une dimension de particule moyenne de l'ordre de 0,05 pm à 15 pm.

6. Composition selon une quelconque des revendications 1 à 5, dans laquelle la composition est sous forme de gel.

7. Composition selon une quelconque des revendications 1 à 6, qui contient au moins un gaz dans les bulles ultrafines tel que sélectionné parmi le groupe constitué d'oxygène, hydrogène, azote, dioxyde de carbone et ozone.

8. Procédé de préparation de la composition de la revendication 1 consistant à
générer à l'aide d'un générateur de bulles ultrafines des bulles ultrafines dans l'eau, lesquelles bulles ultrafines ont une dimension de particule modale ne dépassant pas 500 nm et sont présentes à une densité d'au moins 1 x 10⁶ par millilitre, dans lequel les surfaces des bulles ultrafines sont électriquement chargées pour fournir des potentiels zêta qui sont d'au moins 5 mV en valeur absolue,
dans lequel un médicament qui est une substance évaporative sélectionnée parmi des parfums et des huiles essentielles est dissous ou dispersé dans l'eau avant la génération des bulles ultrafines ou est dispersé dans l'eau après la génération des bulles ultrafines.

9. Procédé selon la revendication 8, dans lequel le médicament est hydrosoluble et le procédé comprend la génération des bulles ultrafines dans une solution du médicament hydrosoluble dans l'eau.

10. Procédé selon la revendication 8, dans lequel le médicament est hydrophobe et le procédé comprend la génération des bulles ultrafines dans une dispersion du médicament hydrophobe dans l'eau.

11. Procédé selon la revendication 8, dans lequel le médicament est hydrophobe et le procédé comprend l'addition du médicament hydrophobe à de l'eau comprenant les bulles ultrafines après la génération des bulles ultrafines dans l'eau.

12. Procédé selon une quelconque des revendications 8 à 11, dans lequel le générateur de bulles ultrafines est un dispositif de cisaillement de mélange gaz-liquide.
